# FASCICULE DE BREVET EUROPEEN

(11) **EP 1 432 812 B1**
(45) Date de publication et mention de la délivrance du brevet: **05.03.2008**
(21) Numéro de dépôt: 02783159.3
(22) Date de dépôt: 13.09.2002
(51) Int. Cl.: C12Q 1/04, C12M 1/12

(54) **DISPOSITIF ET PROCEDE DE CONCENTRATION ET DE DETECTION DE GERMES PATHOGENES A PARTIR DE PRODUITS SANGUINS ET/OU DE LEURS DERIVES**
VORRICHTUNG UND VERFAHREN ZUR KONZENTRATION UND ZUM NACHWEIS PATHOGENER KEIME AUS BLUTPRODUKTEN UND/ODER IHRER DERIVATE
DEVICE AND METHOD FOR CONCENTRATION AND DETECTION OF PATHOGENIC GERMS FROM BLOOD PRODUCTS AND/OR THEIR DERIVATIVES

(30) Priorité: 13.09.2001 FR 0111873
(43) Date de publication de la demande: 30.06.2004
(73) Titulaire: GeneOhm Sciences, Inc., San Diego CA 92121 (US)
(72) Inventeur: HERMET, Jean-Pierre, 92100 Boulogne (FR); BESSON-FAURE, Isabelle, 13400 Aubagne (FR); RIBAULT, Sébastien, 13380 Plan de Cuques (FR); GODFRIN, Yann, 69009 Lyon (FR); MONNOT DES ANGLES, Anne, F-71100 Saint Rémy (FR)
(74) Mandataire: Breese, Pierre
(86) Numéro de dépôt international: PCT/FR2002/003132
(87) Numéro de publication internationale: WO 2003/025207

(56) Documents cités:
- WO-A-01/09370
- WO-A-89/04372
- DE-A- 19 801 090
- US-A- 3 635 798
- US-A- 5 302 512
- US-A- 5 316 731
- US-A- 5 766 552
- US-A- 5 981 294
- PARTHUISOT ET AL: "Evaluation of chemchrome V6 for bacterial viability assrssment in waters" JOURNAL OF APPLIED MICROBIOLOGY, 22 mars 2000 (2000-03-22), pages 370-380,
- RIBAULT ET AL: "Rapid screening method for detection of bacteria in platelet concentrates" JPURNAL OF CLINICAL MICROBIOLOGY, vol. 42, no. 5, mai 2004 (2004-05), pages 1903-1908,
- RIBAULT ET AL: "Detection of bacteria in red blood cell concentrates by the scansystem method" JOURNAL OF CLINICAL MICROBIOLOGY, vol. 43, no. 5, mai 2005 (2005-05), pages 2251-2255,

## Description

La présente invention a pour objet une méthode de concentration de germes pathogènes éventuellement présents dans les produits sanguins ou dérivés, ainsi que la détection desdits germes ainsi concentrés pour effectuer un contrôle de la pathogénicité desdits produits sanguins.

On entend par produit sanguin tant le sang total, comme toute préparation issue du fractionnement de celui-ci, comprenant ou non des composants cellulaires, à titre d'exemple, on entend par produit sanguin les concentrés des globules rouges ou de plaquettes, mais aussi les préparations plasmatiques ou sériques.

La détection de contaminations des produits sanguins et dérivés par différents germes pathogènes, tels que des bactéries, des virus, des moisissures, des levures ou autres, est l'une des grandes difficultés auxquelles sont confrontées de nos jours les autorités responsables de la santé publique, ainsi que les industries de la transfusion sanguine.

Des tests de détection existent, mais ils ne sont pas utilisables en routine à ce jour.

US 3635798 décrit un procédé de concentration de micro-organismes présents dans le sang caractérisé par les étapes suivantes : addition d'un agent d'agrégation pour agréger les cellules sanguines résultant en leur sédimentation, filtration du surnageant pour éliminer les cellules sanguines résiduelles, filtration de ce filtrat pour isoler les bactéries, détection des bactéries.

DE 19801090 décrit un procédé de détection de micro-organismes présents dans le sang comprenant : lyse spécifique des cellules sanguines, filtration pour isoler les bactéries, détection des micro-organismes éventuellement après marquage.

Les principales difficultés présentées par la plupart des tests de détection de tels germes pathogènes, parmi une population ou une sous-population de cellules sanguines, réside dans le fait que la plupart de traitements censés extraire sélectivement les germes pathogènes, provoquent simultanément une élimination de ceux-ci.

Cette élimination conduit presque systématiquement à une sous-évaluation de la présence desdits germes dans le produit sanguin testé, et donc à une augmentation du risque sanitaire.
La demanderesse a donc conçu une nouvelle méthode rapide et sensible pour détecter une contamination d'un produit sanguin ou dérivé, par de tels germes pathogènes.
Le procédé selon l'invention est remarquable en ce qu'il est réalisé directement sur un échantillon issu d'un produit sanguin prélevé chez un sujet, sans traitement ou dilution préliminaire.
Le procédé de détection de germes pathogènes développée par la Demanderesse consiste essentiellement à concentrer sélectivement lesdits germes pathogènes, puis à les détecter, une fois concentrés, par des techniques connues de l'homme du métier.
La concentration sélective des germes pathogènes est effectuée par élimination séquentielle ou simultanée des différentes populations de cellules sanguines présentes dans un échantillon d'un produit sanguin.
Le procédé de concentration de germes pathogènes selon l'invention comporte une première étape de concentration des germes pathogènes consistant à réduire la concentration des populations des cellules sanguines au moyen d'une agrégation sélective de celles-ci, suivie d'une filtration afin de recueillir dans le filtrat les germes pathogènes concentrés non agrégés et de retenir sur le filtre les agrégats de cellules sanguines.

On entend par agrégation, au sens de la présente invention, toute action conduisant à la formation d'agrégats cellulaires, et on entend par agrégat cellulaire tout groupement de cellules comportant plus de deux cellules et dont la taille est supérieure à celle d'une cellule isolée. Au sens de la présente invention, un agrégat peut être obtenu soit par agrégation, telle que l'agrégation des plaquettes subséquente à leur activation, une agglutination, telle que l'agglutination des globules rouges obtenue lors que ceux-ci se trouvent en présence de molécules particulières, soit un rapprochement des cellules induit par un changement de la charge électrostatique de leurs membranes ou encore d'autres mécanismes d'adhésion ou de rapprochement cellulaire conduisant au regroupement de plus de deux cellules.

Selon des modes de mise en oeuvre préférés, l'agrégation des différentes populations de cellules sanguines peut être effectuée à l'aide de composés induisant l'agrégation plaquettaire, ou bien à l'aide de composés induisant l'agglutination spécifique des globules rouges.
On connaît par exemple que, en présence de certains composés, les plaquettes ont la capacité de s'agréger. Ces agrégats peuvent être facilement séparés des germes pathogènes par filtration.
Les globules rouges présentent également quelques propriétés d'agglutination.

Le procédé de concentration de germes pathogènes selon l'invention comporte une deuxième étape de réduction de la concentration des populations des cellules majoritaires dans le sang, à savoir les plaquettes et les globules rouges, consistant à lyser les cellules isolées non agrégées lors de la première étape d'agrégation.

Cette deuxième étape de réduction de la concentration des populations des cellules sanguines permet une réduction de l'ordre de 4 log (depuis 10⁹ à 10⁵ cellules/ml) en ce qui concerne la concentration des plaquettes et de l'ordre de 5 log (depuis 10¹⁰ à 10⁵) en ce qui concerne la concentration des globules rouges.

Plus précisément l'invention a pour objet un procédé de concentration de germes contaminants éventuellement présents dans un produit sanguin comprenant des cellules sanguines, caractérisé en ce qu'il comprend les étapes suivantes :
a) on soumet un échantillon dudit produit sanguin à un traitement d'agrégation des cellules sanguines,
b) on élimine les agrégats formés à l'étape (a) par passage de l'échantillon traité sur un premier filtre laissant passer les germes contaminants mais pas les agrégats de cellules,
c) on lyse sélectivement les cellules résiduelles du filtrat obtenu à l'étape (b),
d) on récupère les germes contaminants par passage du lysat de l'étape (c) sur un second filtre laissant passer des débris cellulaires.
e) on ajoute un agent de marquage des germes contaminants, soit lors de l'agrégation de l'étape (a), soit lors de la lyse de l'étape (c), et
f) on analyse le second filtre pour détecter les germes contaminants marqués éventuellement retenus.

A titre d'exemple d'agent de marquage des germes pathogènes détectables par le procédé de l'invention, on peut citer une solution de marquage comprenant un substrat estérasique tel que le ChemChrome V6.
Aussi, en tant d'agent de marquage des germes pathogènes détectables par le procédé de l'invention, on peut utiliser une solution de marquage comprenant un anticorps marqué ou un marqueur d'acides nucléiques. Préférentiellement, le marqueur sera fluorescent ou couplé à un fluorochrome ou à un enzyme permettant la dégradation d'un substrat rendu ainsi fluorescent, éventuellement la fluorescence sera détectée par une excitation laser.

Le procédé de détection selon l'invention comprend également l'addition d'un agent de perméabilisation des germes contaminants, qui peut être ajouté à au moins l'une des étapes du procédé, soit lors de l'agrégation de l'étape (a), soit lors de la lyse de l'étape (c), soit directement sur le deuxième filtre lors de l'étape (e) d'analyse, soit à plusieurs d'entre elles.
A titre d'exemple d'agent de perméabilisation des germes contaminants, on peut citer le polyéthylènimine, le diacétate de chlorhexidine, le digluconate de chlorhexidine, l'acide d'éthylène diamine tetracétate (EDTA) seul ou en combinaison avec la nisine ainsi que les détergents de type N-Octyl β-D-glucopyranoside, SDS, Tween, triton, Brij, etc.

Selon un mode particulier de mise en oeuvre du procédé de l'invention, les cellules sanguines du produit sanguin sont des plaquettes ou des globules rouges ou un mélange de ceux-ci.

Selon un autre mode particulier de mise en oeuvre du procédé de l'invention, les cellules sanguines du produit sanguin sont des plaquettes et le traitement d'agrégation de l'étape (a) comprend la mise en contact de l'échantillon avec une composition d'agrégation comprenant au moins l'un des agents d'agrégation choisis dans le groupe comprenant :
- un anticorps spécifique d'un antigène plaquettaire,
- un agoniste fort de l'activation plaquettaire, choisi parmi : la thrombine, le TRAP (thrombin receptor activating peptide), la trypsine, le collagène, le thromboxane A2, le PAF (platelet activating factor), le ionophore A23187.
- un agoniste faible de l'activation plaquettaire, choisi parmi ADP, adrénaline, acide arachidonique, facteur Von Willebrand, sérotonine ou épinéphrine.

Avantageusement la concentration en anticorps de type CD9 spécifique d'un antigène plaquettaire dans la composition d'agrégation est comprise entre 0,5 µg/ml et 100 µg/ml, et de préférence elle est comprise entre 5 µg/ml et 40 µg/ml.

Encore avantageusement, la concentration en agoniste fort dans la composition d'agrégation est comprise entre :
- 0,5 UI/ml et 100 UI/ml et de préférence entre 1 UI/ml et 20 UI/ml pour un agoniste de type thrombine,
- 5 µM et 200 µM et de préférence entre 10 et 100 µM pour un agoniste de type TRAP,
- 1 nM et 500 nM et de préférence entre 10 nM et 300 nM pour un agoniste de type trypsine,
- 0,05 µg/ml et 50 µg/ml et de préférence entre 1 µg/ml et 20 µg/ml pour un agoniste de type collagène,
- 0,01 µg/ml et 5 µg/ml et de préférence entre 0,1 et 1 µg/ml pour un agoniste du type thromboxane A2,
- 0,005 mg/ml et 1 mg/ml et de préférence entre 0,05 et 0,5 mg/ml pour un agoniste du type PAF,
- 0,1 µM et 100 µM et de préférence entre 1 µM et 20 µM pour un agoniste du type ionophore A23187.

Avantageusement la concentration en agoniste faible dans la composition d'agrégation est comprise entre :
- 0,5 µM et 100 µM et de préférence entre 1 µM et 20 µM pour un agoniste du type ADP,du type adrénaline ou du type épinéphrine.
- 0,001 mM et 10 mM et de préférence entre 0,01 et 5 pour un agoniste du type acide arachidonique,
- 0,001 mg/ml et 1 mg/ml et de préférence entre 0,01 mg/ml et 0,5 mg/ml pour un agoniste du type facteur Von Willebrand,
- 0,05 µM et 100 µM et de préférence entre 0,01 µM et 50 µM pour un agoniste du type sérotonine.

D'une manière tout à fait préférentielle l'anticorps spécifique d'un antigène plaquettaire, est choisi parmi : un anticorps anti-CD9, CD32, anti-PTA1, CD42, anti-GpIIb/IIIa ou anti-GpIV.

Selon un autre mode particulier de mise en oeuvre du procédé de l'invention, le produit sanguin comprend des globules rouges et le traitement d'agrégation de l'étape (a) comprend la mise en contact de l'échantillon avec une composition d'agglutination comprenant au moins un agent d'agglutination choisi parmi les lectines, le polyéthylènimine, le polyvinylpyrrolidone (PVP), les gélatines, les dextrans ou les polyéthylènes glycols (PEG).

Avantageusement lesdites lectines présentent une activité érythroagglutinine.
Tout préférentiellement les lectines sont choisies parmi les lectines de *Phaseolus vulgaris, Vicia sativa, Vicia faba* ou *Erythrina corallodendron., Lens culinaris, Phytolacca Americana, Triticum vulgaris.*
Avantageusement la concentration de lectine de type *Phaseolus vulgaris* dans la composition d'agglutination est comprise entre 10 µg/ml et 200 µg/ml.

Avantageusement la concentration de polyéthylènimine dans la composition d'agglutination, est comprise entre 0.1% (poids/volume) et 40% (poids/volume)

Tout préférentiellement lesdits dextrans sont choisis parmi le Dextran 70, Dextran 100, Dextran 500, etc.
Avantageusement la concentration de dextran dans la composition d'agglutination est comprise entre 0.1% (poids/volume) et 40% (poids/volume).

Tout préférentiellement lesdits PEG sont choisis parmi les PEG35, PEG, etc.
Avantageusement la concentration de PEG dans la composition d'agglutination est comprise entre 0.05% (poids/volume) et 40% (poids/volume).

Avantageusement la concentration de gélatine dans la composition d'agglutination est comprise entre 0.5% (poids/volume) et 40% (poids/volume).
Tout préférentiellement lesdites PVP sont choisies parmi les PVP-40, PVP-360, etc.
Avantageusement la concentration de PVP dans la composition d'agglutination est comprise entre 0.05% (poids/volume) et 40% (poids/volume).
Avantageusement, la lyse des cellules de l'étape (c) est réalisée avec une solution de lyse comprenant un ou plusieurs détergents choisis dans le groupe comprenant : saponine, SDS, Tween 20, Triton X100, Brij 96, Polidocanol, N-Octyl β-D-glucopyranoside ou Carbonate de sodium.
De préférence, la solution de lyse est constituée d'un mélange de saponine, de Triton X100 et de Tween 20, et tout préférentiellement la solution de lyse comprend de la saponine à une concentration (exprimée en % poids/volume) comprise entre 0,005% et 0,5%, du Triton X100 à une concentration (exprimée en % poids/volume) comprise entre 0,001% et 0,5% du Tween 20 à une concentration comprise entre 0,01% et 1%, du N-Octyl β-D-glucopyranoside à une concentration comprise entre 0,1% et 0,5%.
Avantageusement, la perméabilisation des bactéries est réalisée avec une solution comprenant un ou plusieurs réactifs choisis dans le groupe comprenant : Chlorhexidine (digluconate, diacétate), Polyéthylènimine, N-Octyl β-D-glucopyranoside, Nisin seule ou en combinaison avec de l'EDTA.

De préférence, les agents perméabilisants sont utilisés pour la chlorhexidine à une concentration (poids/volume) comprise entre 0,0001% (poids/volume) et 0,1% (poids/volume), pour le polyéthylènimine une concentration comprise entre 5 µg/ml et 120 µg/mL, pour le N-Octyl βD-glucopyranoside à une concentration comprise entre 0,1% (poids/volume) et 0,5% (poids/volume) et pour la Nisin entre 0.1 µg/mL et 0,5 µg/mL seule ou en combinaison avec de l'EDTA à une concentration comprise entre 1 mM et 10 mM.

Le procédé de l'invention peut être utilisé pour concentrer et détecter de nombreux germes contaminants des produits sanguins, parmi lesquels on peut citer des bactéries aérobies ou anaérobies, des moisissures, des levures, des spores de bactéries vivantes et/ou mortes.

Avantageusement la taille des pores du premier filtre mis en oeuvre dans le procédé de l'invention est comprise entre 2 µm et 20 µm.

Avantageusement la taille des pores du second filtre mis en oeuvre dans le procédé de l'invention est comprise entre 0,2 µm et 2 µm.

Avantageusement la détection des germes contaminants de l'étape (e) du procédé de l'invention est réalisée dans un dispositif clos.

Tout préférentiellement, les germes contaminants capables d'être concentrés selon le procédé de l'invention, sont choisis parmi le groupe des bactéries aérobies ou anaérobies, des moisissures, des levures ou des spores de bactéries vivantes et/ou mortes, la taille des pores du premier filtre est comprise entre 2 µm et 20 µm, et la taille des pores du second filtre est comprise entre 0,2 µm et 2 µm.

D'autres avantages et caractéristiques de l'invention apparaîtront à la lecture des exemples qui suivent et des figures en annexe dans lesquelles :
- la figure 1 illustre le dénombrement des plaquettes après avoir mis en contact les concentrés plaquettaires avec différentes concentrations de thrombine.
- La figure 2 illustre le dénombrement des plaquettes après agrégation en présence d'ADP pour 2échantillons plaquettaires.
- La figure 3 montre le dénombrement des plaquettes en présence d'anticorps CD9 (clone SN4) pour 4 échantillons plaquettaires.
- La figure 4 montre les résultats du dénombrement des plaquettes en présence de doses croissantes d'anticorps CD9 (clone 6B1) utilisé sur 3 mL de concentré plaquettaire.
- La figure 5 montre la courbe dose réponse obtenue en présence de concentrations croissantes d'anticorps CD9 (clone SN4).
- La figure 6 illustre l'agglutination de globules rouges en présence de la lectine *Phaseolus vulgaris* utilisée à une concentration de 200 µg/ml pour 3 échantillons de concentrés de globules rouges.
- La figure 7 montre les effets de la solution de lyse sur la récupération de différents germes pathogènes dans des cultures pures.
- La figure 8 montre l'effet de la solution de lyse sur la numération plaquettaire de deux échantillons de plaquettes.
- La figure 9 illustre l'effet de la lyse sur la numération érythrocytaire de deux échantillons différents de concentrés de globules rouges.

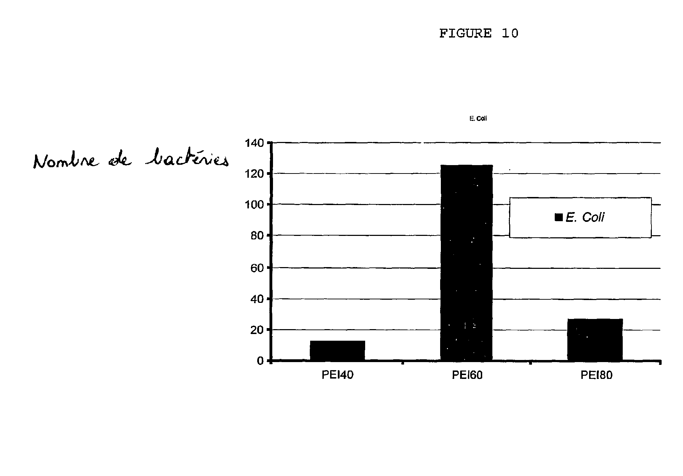

- La figure 10 illustre le dénombrement des bactéries Escherichia coli au sein d'un échantillon plaquettaire en utilisant le polyéthylène imine (PEI) en tant qu'agent perméabilisant pour augmenter la pénétration du marqueur.
- La figure 11 illustre l'effet de la concentration en N-octyl β D-glucopyranoside dans la solution de lyse sur des cultures de bactéries pures.

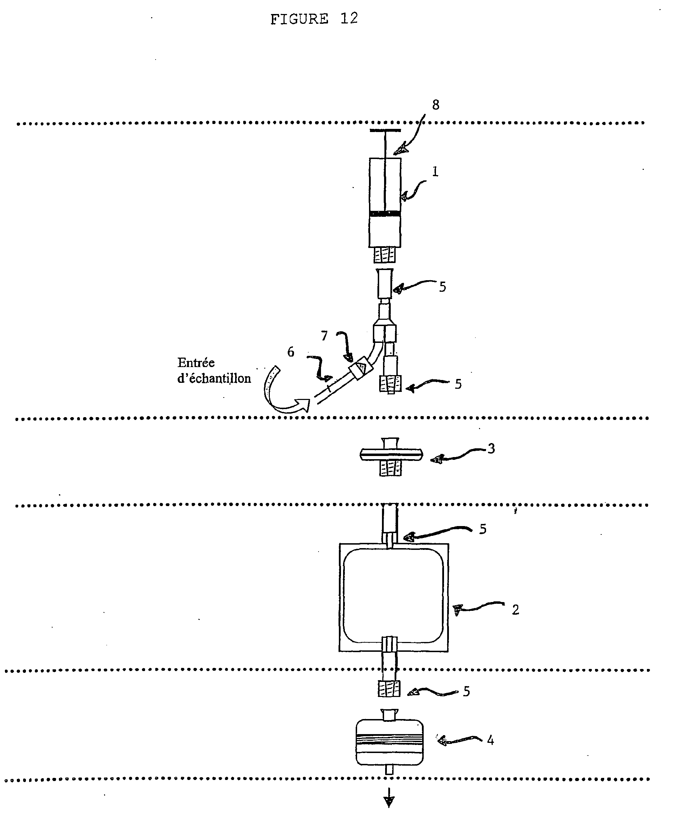

- La figure 12 illustre une mise en oeuvre particulière du dispositif de concentration des germes pathogènes, comprenant:

- un premier réservoir (1) étanche et stérile,
- un deuxième réservoir (2) étanche et stérile,
- un premier filtre stérile (3) placé entre le premier réservoir (1) et le deuxième réservoir (2),
- un deuxième filtre stérile (4) placé en aval du deuxième réservoir (2)
- des moyens de connexion (5) étanches et stériles placés, entre le premier réservoir (1) et le premier filtre (3), entre le premier filtre (3) et le deuxième réservoir (2) et entre le deuxième réservoir (2) et le deuxième filtre (4).
- des moyens de connexion (6) étanches et stériles pour relier la poche contenant le produit sanguin au premier réservoir (1).
- une valve anti-retour (7) placée dans la connexion (6) reliant la poche contenant le produit sanguin au premier réservoir 1.
- un système d'aspiration de l'échantillon (8).

### RESULTATS EXPERIMENTAUX

### I. CONCENTRATION DE GERMES PATHOGENES AU MOYEN D'UNE ETAPE D'AGREGATION

### I.1 Agrégation des plaquettes.

### Exemple 1 . Agrégation avec un agoniste fort: la thrombine.

La thrombine est un agoniste fort de l'agrégation plaquettaire.
Dans les travaux expérimentaux menés par la Demanderesse dans le cadre de la présente invention, des solutions de thrombine (référence T8885 Sigma) ont été préparées : à une concentration de 100 UI/ml lorsqu'elle est utilisée à raison de 10 UI/test et sous forme de solution diluée (100 µl de solution mère de thrombine additionnée de 900 µl de tampon PBS), lorsqu'elle est utilisée à raison de 1 UI/test.
L'agrégation des plaquettes par l'intermédiaire de la thrombine comporte les étapes suivantes :
- On place dans un tube 160 µl de concentré plaquettaire auxquels on ajoute 20 µl de tampon PBS et 20 µl de thrombine
- On agite manuellement les tubes durant 5 minutes à température ambiante.
- On ajoute audits tubes 800 µl de tampon PBS.
- On filtre le contenu des tubes sur un filtre de porosité 11 µm.
- On effectue des dilutions en série 1/20 à 1/10 à partir des filtrats.
- On filtre 100 µl de chaque dilution du filtrat sur une membrane CB04.
- On effectue un marquage estérasique.
- On compte les plaquettes éventuellement retenues sur la membrane.

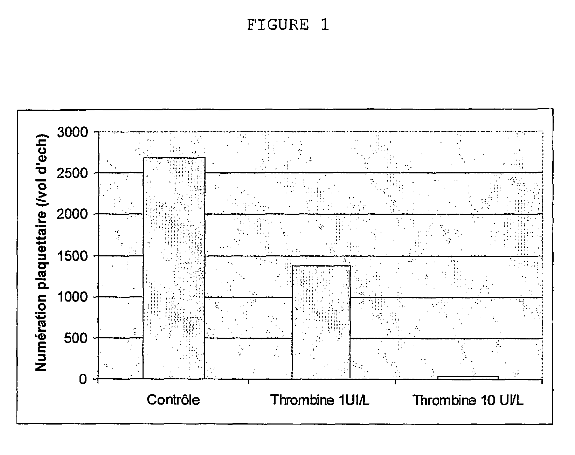

Le tableau 1 ci-dessous illustre les résultats obtenus et représente le nombre d'agrégats plaquettaires obtenus en présence de thrombine utilisée à deux concentrations différentes et la figure 1 en annexe illustre graphiquement ces résultats.

**Tableau 1**

| | Contrôle | Thrombine 1 UI | Thrombine 10 UI |
|---|---|---|---|
| PC 6 | 1584 | 374 | |
| | 1535 | 490 | |
| PC 6 | 2622 | 1358 | 44 |
| | 2737 | 1399 | 30 |

### Exemple 2: Agrégation des plaquettes avec de la thrombine en présence de germes pathogènes.

Les travaux expérimentaux mis en oeuvre afin d'évaluer l'agrégation des germes pathogènes en présence de thrombine comportent les étapes suivantes :
- Une cryosphère de *E. Coli* est introduite dans un tube de 9 ml de bouillon tryptone soja et incubée à 37°C durant 18-24 heures.
- On ajoute à 160 µl de concentré plaquettaire 20 µl de la suspension de *E.coli* et 20µl de thrombine.
- on agite manuellement le tube durant 5 minutes à température ambiante.
- On ajoute 800 µl de tampon PBS,
- On filtre à travers un filtre de porosité 11 µm,
- on effectue des dilutions en série : 1/20 et 1/10 et 1/10,
- On filtre 100 µl de l'échantillon, sur une membrane CB04
- On effectue un marquage estérasique
- On effectue la détection.

Le tableau 2 ci-dessous montre l'agrégation des concentrés plaquettaires avec de la thrombine en présence de *E.coli.*

**Tableau 2**

| Germes pathogènes | Préparation Plaquettes | Thrombine | Résultats Dénombrement |
|---|---|---|---|
| *E. coli* | - | - | 1921 |
| | - | - | 1999 |
| *E. coli* | PC 6 | - | 657 |
| | | | 763 |
| *E. coli* | PC 6 | 1 UI | 140 |
| | | | 167 |
| *E. coli* | PC 6 | 10 UI | 109 |
| | | | 122 |

Après addition de la thrombine, un caillot apparaît presque instantanément.

### Exemple 3: Agrégation des plaquettes en présence d'un agoniste faible: l'ADP.

De l'ADP (SIGMA) est utilisé à une concentration de 200 µM dans de l'eau distillée.
Les travaux expérimentaux mis en oeuvre afin d'évaluer l'agrégation des plaquettes en présence d'ADP comportent les étapes suivantes :
- On introduit dans un tube 400 µl de concentré plaquettaire, auxquels on additionne 50 µl de tampon PBS et 50 µl d'ADP,
- Les tubes sont agités manuellement durant 5 minutes à température ambiante,
- On y ajoute 500 µl de tampons PBS,
- On effectue des dilutions en série de 1/20 et 1/10 et 1/10
- On filtre 100 µl de l'échantillon sur une membrane CB04,
- On effectue un marquage estérasique, et
- On effectue la détection.

Les résultats illustrés sur la figure 1 montrent que, en présence d'ADP, la concentration de plaquettes est réduite d'environ 50% à environ 90%.

### Exemple 4 : Agrégation plaquettaire avec de l'ADP en présence de germes pathogènes.

L'agrégation des plaquettes en présence d'ADP comporte les étapes suivantes :
- Une cryobille de *Staph epidermidis* est introduite dans un tube de 9 ml de bouillon tryptone soja et incubée à 37°C durant 18-24 heures.
- Sur 400 µl de concentré plaquettaire on ajoute 50 µl de la suspension de *Staph epidermidis* et 50 µl d'ADP,
- Les tubes sont agités manuellement durant 5 minutes à température ambiante
- On y ajoute 500 µl de tampon PBS,
- On effectue une filtration sur un filtre de porosité 5 µm,
- On effectue des dilutions en série de 1/20 et 1/10 et 1/10,
- On filtre 100 µl de l'échantillon sur une membrane CB04
- On effectue un marquage estérasique,
- On effectue la détection.

Les germes pathogènes sont ensemencés à une concentration élevée de manière à augmenter un possible effet de piégeage. L'ADP est ajouté aux plaquettes et aux bactéries à une concentration de 10 µM.

**Tableau 3**

| **Germe pathogène** | **Préparation plaquettaire** | **Agrégant** | **Résultats** | **Concentration bactéries/ml** |
|---|---|---|---|---|
| *Staph. epidermidis* | PC 6 | néant | 590 605 | 6,0E+07 |
| *Staph. epidermidis* | PC 6 | ADP 10µM | 492 441 | 4,7E+07 |

Les résultats obtenus, représentés sur le Tableau 3 ci-dessus montrent que 74% de bactéries sont récupérées après l'étape d'agrégation

### Exemple 5 : Agrégation des plaquettes en présence d'un anticorps CD9.

Il est connu que l'anticorps CD9 induit l'activation des plaquettes et par conséquent leur agrégation. Les tests décrits ont été réalisés avec deux clones CD9, le clone SN4 (Ancel, ref 156-020, concentration 100 µg/ml) et le clone 6B1 (Hemosystem).

Le procédé d'agrégation sélective mis en oeuvre avec cet anticorps CD9 comporte les étapes suivantes :
- Sur 400 µl de concentré plaquettaire on ajoute 50 µl de tampon PBS et 50 µl d'anticorps CD9,
- Les tubes sont agités manuellement durant 5 minutes à température ambiante
- On y ajoute 500 µl de tampon PBS,
- On effectue une filtration sur un filtre de porosité 5 µm
- On effectue des dilutions en série de 1/20 et 1/10 et 1/10,
- On filtre 100 µl de l'échantillon sur une membrane CB04,
- On effectue un marquage estérasique,
- On effectue la détection.

L'anticorps CD9 (clone SN4) a été utilisé en présence des plaquettes à une concentration finale de 10 µg/ml.

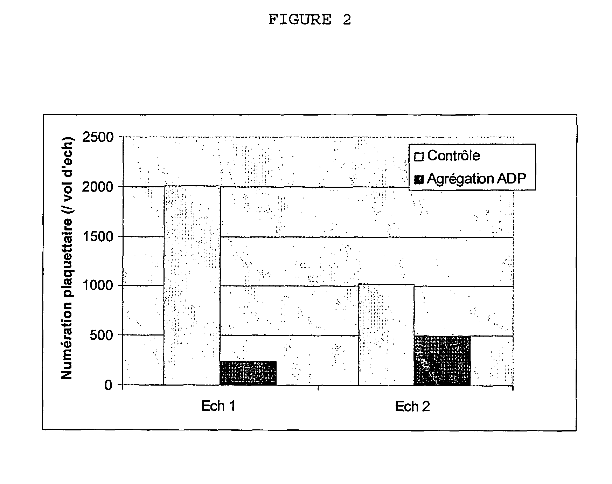

La figure 2 en annexe illustre l'agrégation des plaquettes obtenue en présence d'anticorps CD9 (clone SN4).
Afin d'évaluer la concentration optimale de l'anticorps CD9 nécessaire pour l'agrégation plaquettaire, une courbe dose-réponse a été établie.
Méthode :
- Sur 400 µl de concentré plaquettaire placés dans des tubes on ajoute différentes dilutions d'anticorps, avec des concentrations finales allant de 0 à 20 µg/ml en anticorps CD9.
- Les tubes sont agités manuellement durant 5 minutes à température ambiante
- On y ajoute 500 µl de tampon PBS,
- On effectue une filtration sur un filtre de porosité 5 µm
- On effectue des dilutions en série : 4 dilutions 1/10
- On filtre 100 µl de l'échantillon sur une membrane CB04
- On effectue un marquage estérasique,
- On effectue la détection

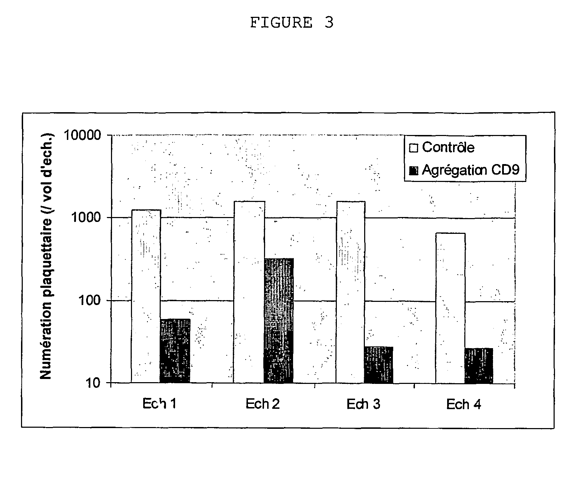

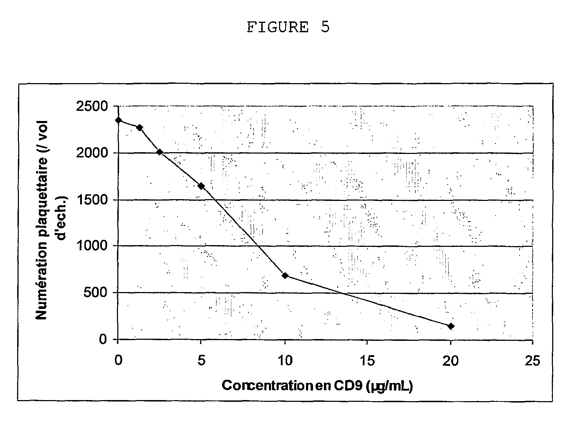

Les figures 3 et 5 montrent respectivement les résultats du dénombrement des plaquettes agrégées en présence de doses croissantes d'anticorps CD9 (clone SN4) et la courbe dose-réponse ainsi obtenue.
Le dénombrement des plaquettes résiduelles est effectué avec l'Analyseur.
L'agrégation est dose-dépendante. Pour augmenter l'effet d'agrégation, la concentration en CD9 a été augmentée autant que possible. Cependant un compromis a été établi pour la détection des bactéries, et l'on a déterminé que la concentration plaquettaire peut être réduite de 1 à 2 Log en utilisant une concentration d'environ 10 µg/ml d'anticorps CD9.

### Exemple 5 bis :

Afin d'évaluer la concentration optimale de l'anticorps CD9 (clone 6B1) nécessaire pour l'agrégation plaquettaire, une expérience du type dose-réponse a été établie

Méthode :
- Sur 3 mL de concentré plaquettaire placés dans des tubes on ajoute différentes dilutions d'anticorps, avec des concentrations finales allant de 2,5 µg/mL à 40 µg/mL en anticorps CD9.
- Les tubes sont agités durant 15 minutes à température ambiante
- On effectue une filtration sur un filtre de porosité 5 µm
- On effectue la numération des plaquettes comme décrit ci-dessus.

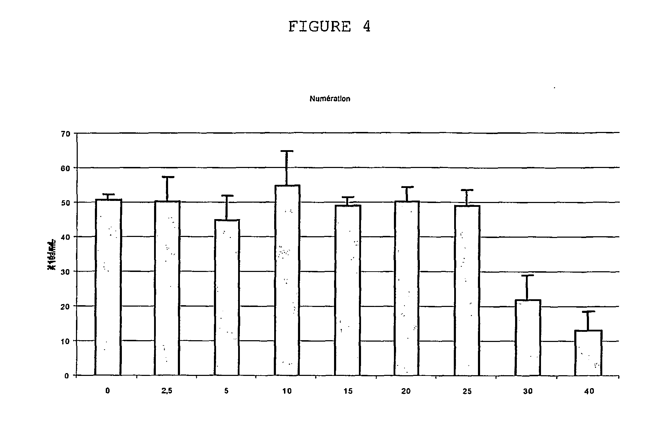

   La figure 4 en annexe montre les résultats du dénombrement des plaquettes agrégées en présence de doses croissantes d'anticorps CD9 (clone 6B1).

### Exemple 6 : Agrégation plaquettaire avec l'anticorps CD9 en présence de bactéries.

Méthode :
- Une cryobille de Staph epidermidis est introduite dans un tube de bouillon tryptone soja et incubée à 37°C durant 18-24 heures.
- Sur 400 µl de concentré plaquettaire placés dans des tubes on ajoute 50 µl de la suspension de Staph epidermidis et 50 µl de CD9,
- Les tubes sont agités manuellement durant 5 minutes à température ambiante
- On y ajoute 500 µl de tampon PBS,
- On effectue une filtration sur un filtre de porosité 5 µm
- On effectue des dilutions en série : 4 dilutions 1/10
- On filtre 100 µl de l'échantillon sur une membrane CB04
- On effectue un marquage estérasique.
- On effectue la détection

Le tableau 4 ci-dessous illustre l'agrégation des plaquettes avec du CD9 en présence de bactéries.

**Tableau 4**

| Bactéries | | CD9 | Résultat | % de récupération |
|---|---|---|---|---|
| Staph.epidermidis | PC 10 | - | 46 | |
| | | - | 44 | |
| Staph.epidermidis | PC 10 | + Agitation | 43 | 93 |
| | | | 48 | |
| E. coli | PC 10 | | 218 | |
| | | | 243 | |
| E. coli | PC 10 | CD 9 + Agitation | 166 | 64 |
| | | | 148 | |

Ces résultats montrent que les bactéries Staph epidermidis ont été bien récupérées. Pour E.Coli le dénombrement est réduit de 36%.

### I.2. Agglutination de globules rouges.

Les lectines sont des glycoprotéines d'origine non-immune qui agglutinent les cellules et/ou précipitent des complexes carbohydrates. Ces molécules se lient couramment à des carbohydrates spécifiques.
Dans le cadre de ces travaux expérimentaux, deux lectines ont été utilisées :
- Phaseoulus vulgaris PHA-E
- Vicia Sativa

### Exemple 1 : Agglutination de globules rouges avec des lectines.

La lectine Phaseolus vulgaris PHA-E (Sigma) a été utilisée à une concentration de 2 mg/ml dans du PBS.
La lectine Vicia Activa (Sigma) a été utilisée à la concentration de 1 mg/ml.
Une évaluation rapide des deux lectines a permis de vérifier qu'il n'y a pas d'agglutination des globules rouges avec Vicia sativa. D'autre part Phaseolus vulgaris induit une agglutination rapide et efficace.

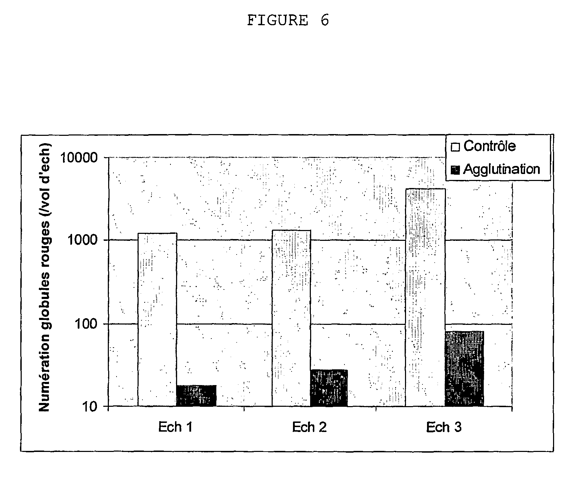

Méthode :
- 400 µl de globules rouges sont placés dans des tubes auxquels on ajoute 50 µl de PBS et 50 µl de Phaseolus vulgaris,
- Les tubes sont agités manuellement durant 5 minutes à température ambiante
- On y ajoute 500 µl de tampon PBS,
- On effectue une filtration sur un filtre de porosité 5 µm,
- On effectue des dilutions en série : 4 dilutions 1/10
- On effectue un marquage avec un anticorps anti-glycophorine-PE.
- On effectue la détection
   La figure 6 en annexe montre l'agglutination des globules rouges obtenue en présence de Phaseolus vulgaris.
   Dans ce test, Phaseolus vulgaris a été utilisée à une concentration de 200 µg/ml.
   On observe une diminution reproductible de deux logs de la concentration en globules rouges en présence de Phaseolus vulgaris.

### Exemple 2 : Agglutination de globules rouges en présence de bactéries.

Les tests préliminaires ont montré qu'il n'y a pas d'interaction entre la lectine Phaseolus et les bactéries.
Méthode
Une cryobille de E.coli est introduite dans un tube de 9 ml de bouillon tryptone soja et incubée à 37°C durant 18-24 heures.
- On mélange dans des tubes, 400 µl de PBS, 50 µl de E.coli (culture pure) et 50 µl de Phaseolus vulgaris,
- Les tubes sont agités manuellement durant 5 minutes à température ambiante
- On y ajoute 500 µl de tampon PBS,
- On effectue une filtration sur un filtre de 5 µm,
- On effectue des dilutions en série : 4 dilutions 1/10,
- On filtre 100 µl de l'échantillon sur une membrane CB04,
- On effectue un marquage estérasique,
- On effectue la détection.
   Les résultats obtenus, en utilisant une concentration Phaseolus vulgaris de 200µg/ml sont illustrés dans le Tableau 5 ci-dessous.

**Tableau 5**

| **Bactéries** | **Globules rouges** | **Lectine (Concentration)** | **Dénombrement** | **% récupération** |
|---|---|---|---|---|
| *E. coli* | RBC 9 | | 662 | |
| NCTC 9001 | | | 604 | |
| *E. coli* | RBC 9 | Phaseolus | 544 | |
| NCTC 9001 | | 200 µg/ml | 579 | 91 |

Ces résultats montrent que 91% des bactéries sont détectées après l'étape d'agglutination et qu'après cette étape d'agglutination, la concentration en globules rouges est réduite de deux logs alors que la souche E. coli est encore bien récupérée.

### II. CONCENTRATION DE GERMES PATHOGENES AU MOYEN D'UNE ETAPE DE LYSE.

Dans le cadre de la présente invention, la demanderesse a effectué une mise au point des différentes techniques de lyse sélective permettant l'élimination des cellules sanguines sans affecter la concentration des bactéries éventuellement présentes dans les échantillons à analyser.
Suite à quelques études préalables, la Demanderesse a constaté que certaines bactéries sont résistantes en présence de détergents tels que le Triton X100 et a déterminé la concentration optimale de détergents avec laquelle le pourcentage de récupération de bactéries est optimal.

### Exemple 1 : Effet de la formulation de la solution de lyse sur des cultures de bactéries pures.

Méthode
- Les souches sont conservées dans un tube de 9 ml de bouillon tryptone soja et incubées à 37 °C durant 18-24 heures.
- Des dilutions en série (1/10) ont été effectuées dans du tampon PBS jusqu'à 10⁻⁵
- Un millilitre de la dernière dilution est traité avec 9 ml de la solution de lyse (0,01% (poids/volume) de saponine, 0,1% (poids/volume) de Tween et 0,001% (poids/volume) Triton X 100 durant 15 minutes
- 100 µl de l'échantillon sont filtrés sur une membrane CB04
- Un marquage estérasique est effectué selon les indications figurant en annexe 1
- On effectue la détection.

Les résultats de ces différents essais sont illustrés dans le tableau 6 ci-dessous, dans lequel sont exprimés les différents pourcentages de récupération des bactéries obtenus.

**Tableau 6**

| **Souche** | **Contrôle** | **Échantillon lysé** | **% de récupération** |
|---|---|---|---|
| *E. coli* | 132 | 153 | 98 |
| | 145 | 119 | |
| *Bacillus cereus* | 465 | 61 | 11 |
| | 572 | 54 | |
| *E. coli* | 52 | 44 | 83 |
| | 54 | 44 | |
| *Staph. epidermidis* | 2952 | 3034 | 101 |
| | 3069 | 3020 | |
| *E. aerogenes* | 841 | 880 | 110 |
| | 802 | 925 | |
| *Ps. aeruginosa* | 261 | 82 | 31 |
| | 215 | 66 | |
| *Staph. aureus* | 105 | 126 | 125 |
| | 94 | 122 | |
| *P. mirabilis* | 729 | 1129 | 139 |
| | 906 | 1151 | |
| *S. typhymurium* | 608 | 820 | 133 |
| | 600 | 787 | |
| *Serratia marcescens* | 1848 | 1826 | 102 |
| | 1775 | 1860 | |
| *C. amycolatum* | 1103 | 1512 | 156 |
| | 1167 | 2035 | |
| *K. pneumoniae* | 72 | 79 | 99 |
| | 82 | 73 | |
| *P. fluorescens* | 4039 | 4873 | 125 |
| | 3951 | 5105 | |
| *Streptococcus bovis* | 3074 | 1848 | 64 |
| | 2939 | 1978 | |
| *Y.enterocolitica* | 10218 | 9888 | 99 |
| | 10321 | 10526 | |

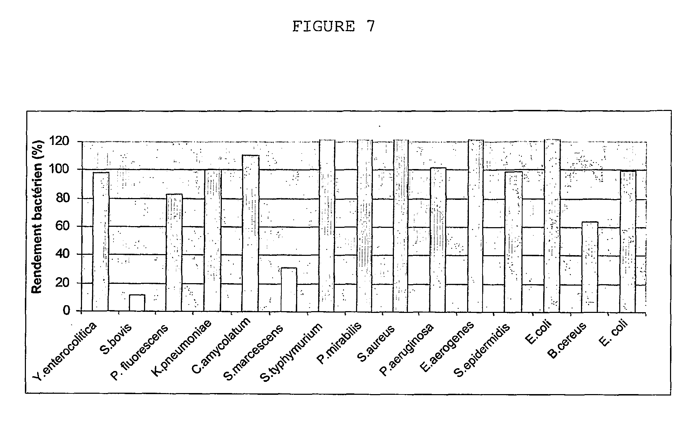

Ces résultats sont illustrés sur la figure 7 en annexe.
La plupart des souches ne sont pas affectées par la solution de lyse: les pourcentages de récupération sont cohérents avec les prévisions prédéfinies, entre 95 et 115%, sauf pour *Ps. aeruginosa* et *Bacillus cereus.*

### Exemple 2 : Effet de la formulation de la solution de lyse sur les bactéries ensemencées dans des plaquettes.

De manière à simuler une contamination, deux souches ont été adaptées à la croissance dans des concentrés plaquettaires.

Méthode
- Les souches *Bacillus cereus* et *Staph. aureus* ont été ensemencées dans des plaquettes dans des tubes de 50 ml en utilisant une concentration de 10⁵ cellules dans 20 ml de plaquettes.
- Ces tubes de 50 ml sont conservés dans l'incubateur de plaquettes à 22 °C durant plusieurs jours,
- Un millilitre de plaquettes ensemencées est dilué avec 9 ml de la solution de lyse,
- La lyse est effectuée durant 15 minutes à température ambiante,
- 100 µl de l'échantillon sont filtrés sur une membrane CB047,
- Les bactéries sont marquées avec un substrat stéarique comme indiqué en annexe 1,
- La membrane est balayée avec l'Analyseur.

Les résultats du dénombrement des bactéries après la lyse sont illustrés dans le tableau 7 ci-dessous.

**Tableau 7**

| | | **Contrôle** | **Lysés** |
|---|---|---|---|
| Bacillus cereus | | 16 | 14 |
| | | 24 | 27 |
| | bactéries/ml | 2,0E+06 | 2,1E+06 |
| Staph. aureus | | 1966 | 1885 |
| | | 2046 | 1901 |
| | bactéries/ml | 2, 0E+08 | 1,9E+08 |

Les bactéries ont été ensemencées à une concentration initiale de 5 10³ cellules/ml et sont détectées plusieurs jours plus tard à des concentrations de l'ordre de 10⁶ à 10⁹ cellules /ml.

Il en résulte de ces expériences, que les bactéries qui se sont développées dans les plaquettes n'ont pas été affectées par la solution de lyse.

### Exemple 2b : Effet de la formulation de la solution de lyse sur des cultures de bactéries pures.

Méthode
- Les souches sont conservées dans un tube de 9 ml de bouillon tryptone soja et incubées à 37 °C durant 18-24 heures.
- Après détermination du nombre de bactéries par marquage estérasique, 3000 bactéries sont inoculées dans 3 mL de PBS
- Les 3mL sont traités avec la solution de lyse (NOG 0.25% à 2%) pendant 20 minutes
- La totalité de l'échantillon est filtrée sur une membrane CB04
- Le dénombrement est effectué en cytométrie en phase solide

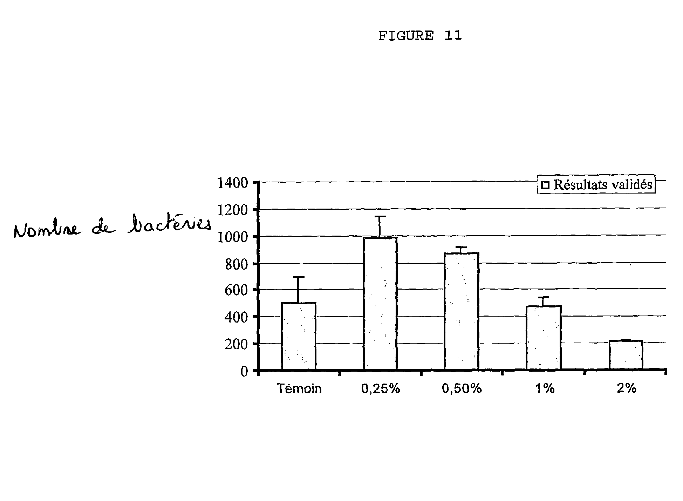

Les résultats obtenus sont illustrés figure 11 en annexe.

### Exemple 3 : Effet de la formulation de la composition de lyse sur les globules rouges.

Méthode
- Un millilitre de globules rouges est dilué dans 9 ml de solution de lyse ou 9 ml de PBS (contrôle).
- la lyse est effectuée durant 15 minutes à température ambiante
- les échantillons lysés ou non sont analysés à l'aide d'un compteur cellulaire.

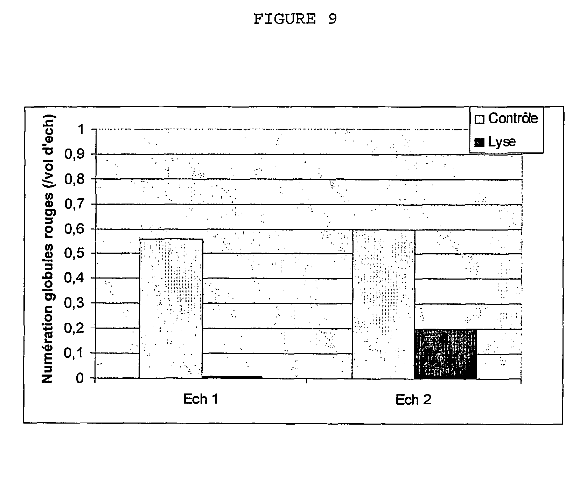

La figure 9 montre les résultats obtenus sur la préparation de globules rouges lysée par rapport à une préparation de globules rouges non lysée.

### Exemple 4 : Effet de la formulation de la composition de lyse sur les plaquettes.

La reproductibilité de l'efficacité de la solution de lyse a été testée. Des échantillons différents de plaquettes ont été lysés et analysés.
Méthode:
- Un millilitre de plaquettes est dilué dans 9 ml de solution de lyse
- la lyse est effectuée durant 15 minutes à température ambiante
- Des dilutions en série (1/10) ont été effectuées dans du tampon PBS jusqu'à 10⁻⁵
- 100 µl de l'échantillon sont filtrés sur une membrane CB04.
- les microorganismes sont marqués avec un substrat estérasique
- la membrane est balayée avec l'analyseur.

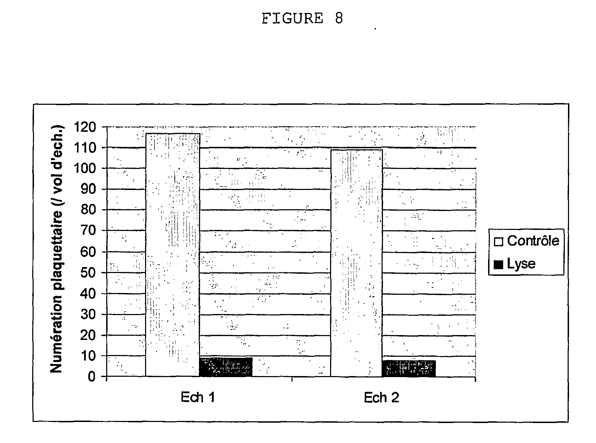

La figure 8 en annexe illustre les résultats de la lyse obtenue avec différents échantillons de plaquettes.

### III. CONCENTRATION DE GERMES PATHOGENES AU MOYEN DE DEUX ETAPES D'AGREGATION ET DE LYSE.

La préparation de l'échantillon par concentration des germes pathogènes en deux étapes comprend :
1) L'Agrégation ou agglutination spécifique des cellules du produit sanguin.
2) La Lyse spécifique des cellules du produit sanguin.

### Exemple 1: Séparation spécifique des plaquettes

Méthode :
- Une cryobille de E.coli est introduite dans un tube de 9 ml de bouillon tryptone soja et incubée à 37°C durant 18-24 heures.
La première étape d'agrégation est effectuée comme suit :
- On mélange dans des tubes, 1 ml de concentré plaquettaire, 50 µl de E.coli (culture pure) et 100 µl de CD 9,
- Les tubes sont agités manuellement durant 5 minutes à température ambiante.
La deuxième étape de lyse est alors effectuée :
- On ajoute 900 µl de tampon de lyse à 100 µl du filtrat après agrégation,
- On maintient l'ensemble 15 minutes à température ambiante,
- On effectue des dilutions en série : 4 dilutions 1/10
- On filtre 100 µl de l'échantillon ainsi obtenu sur une membrane CB04,
- On effectue un marquage des bactéries et des plaquettes avec un substrat estérasique (Annexe 1)
- On dénombre les bactéries et les plaquettes avec l'Analyseur.

Les résultats obtenus en effectuant le procédé de concentration des bactéries en deux étapes sont illustrés dans le tableau 8 ci-dessous.

**Tableau 8**

| **bactéries** | **Concentré plaquettaire (PC)** | **Agent agrégant** | | **Dénombrement bactéries** | **% récupération bactéries** |
|---|---|---|---|---|---|
| *E. coli* | PC | | | 337 | |
| NCTC 9001 | | | | 313 | |
| *E. coli* | PC | | Lyse | 302 | 86% |
| NCTC 9001 | | | | 256 | |
| *E. coli* | PC | CD9 | | 266 | 66% |
| NCTC 9001 | | | | 161 | |
| E. coli | PC | CD9 | Lyse | 157 | 45% |
| NCTC 9001 | | | | 138 | |

### Exemple 2. Séparation spécifique des globules rouges.

Méthode :
- Une cryobille de E.coli est introduite dans un tube de 9 ml de bouillon tryptone soja et incubée à 37°C durant 18-24 heures.
L'étape d'agglutination est effectuée comme suit :
- On mélange dans des tubes, 1 ml de globules rouges, 50 µl de E.coli (culture pure) et 125 µl de lectine,
- Les tubes sont agités manuellement durant 5 minutes à température ambiante
- On effectue une filtration sur un filtre de porosité 5 µm.
La deuxième étape de lyse est alors effectuée :
- On ajoute 900 µl de tampon de lyse à 100 µl du filtrat après agglutination,
- On maintient l'ensemble 15 minutes à température ambiante,
- On effectue des dilutions en série : 4 dilutions 1/10
- On filtre 100 µl de l'échantillon ainsi obtenu sur une membrane CB04,
- On effectue un marquage des bactéries avec un substrat estérasique et des globules rouges avec un anticorps anti-Glycophorine-PE (Annexe 2)
- On dénombre les bactéries et les globules rouges avec l'Analyseur.

Le tableau 9 ci-dessous montre les résultats obtenus avec le dénombrement des bactéries effectué après l'étape d'agglutination des globules rouges avec la lectine suivie de l'étape de lyse.

**Tableau 9**

| | | | | **Dénombrement des bactéries** | **récupération des bactéries (%)** | **Dénombrement des globules rouges** |
|---|---|---|---|---|---|---|
| *E*. *coli* | Globules rouges 10 | | | 441 | | 3582 |
| NCTC 9001 | | | | 449 | | 4188 |
| *E. coli* | Globules rouges 10 | Lectine | Lyse | 261 | 68 | 161 |
| NCTC 9001 | | | | 348 | | 180 |

Après agglutination la concentration en globules rouges a été réduite de 1,5 log.
Soixante-huit (68) % des bactéries sont récupérées après ces deux étapes.

### IV. AGENTS DE MARQUAGE

### Exemple 1. Substrat estérasique ChemChrome V6

Des solutions de marquage peuvent être préparées à partir d'un substrat estérasique et utilisées dans le procédé de détection de l'invention selon le protocole suivant
a) Préparation
   - 10 µl du substrat estérasique ChemChrome V6 par millilitre de tampon ChemSol B16 (500 µl de solution de marquage par membrane)
   - cette solution est conservée à 4°C à l'abri de la lumière au maximum 4 heures.
b) Utilisation
   Introduire un tampon de marquage dans une boîte de Pétri de 33 mm de diamètre.
   Distribuer 500 µl de la solution de marquage sur le tampon.
   Placer la membrane CB04 sur la rampe de filtration. Filtrer 100 µl de l'échantillon à analyser.
   Placer la membrane sur le tampon.
   Incuber durant 15 minutes à 37°C.

### Exemple 2. Anticorps marqué.

Aussi, une solution de marquage comprenant un anticorps marqué peut être utilisée dans le procédé de détection de l'invention selon le protocole suivant
- Prélever 90 µl d'une dilution de l'échantillon,
- Ajouter 10 µl de l'anticorps anti-glycophorine-PE,
- Vortexer et incuber durant 15 minutes à température ambiante à l'abri de la lumière,
- Ajouter 900 µl de tampon PBS,
- Placer la membrane CB04 dans la rampe de filtration,
- Filtrer sous vide 100 µl de la solution à analyser,
- Analyser la membrane dans l'Analyseur en inversant les câbles primaire et tertiaire de l'Analyseur.

### Exemple 3 : Intérêt de l'ajout d'un agent de perméabilisation des bactéries pour améliorer la pénétration du marqueur.

Méthode
- Les souches sont conservées dans un tube de 9 ml de bouillon tryptone soja et incubées à 37 °C durant 18-24 heures.
- Après détermination du nombre de bactéries par marquage estérasique, 3000 bactéries sont inoculées dans 3 mL de concentré plaquettaire
- Les 3mL sont traités avec 1 ml de la solution d'agrégation (CD9 :clone 6B1: 30µg/mL, Picogreen 1/2000, PEI 40µg/mL à 80µg/mL) durant 40 minutes
- L'échantillon est filtré au travers d'un filtre de porosité 5 µm
- L'échantillon est incubé dans la solution de lyse (Chlorhexidine 5.10⁻³ %, NOG 0,5%, Nisin 0,2 µg/mL, EDTA 5 mM)) pendant 20 minutes
- La totalité de l'échantillon est filtrée sur une membrane CB04
- Le dénombrement est effectué au moyen d'un analyseur cytomètre en phase solide

### V. CONCLUSIONS

Les options techniques pour atteindre les meilleures conditions de préparation des germes pathogènes ont été définies à l'aide de ses travaux expérimentaux.

En ce qui concerne les concentrés plaquettaires ces options techniques comportent :
1) Etape d'agrégation avec par exemple un anticorps activateur plaquettaire tel que CD9,
2) Etape de lyse cellulaire avec une combinaison des détergents tels que saponine, Tween 20 et Triton X 100.

En ce qui concerne les concentrés de globules rouges :
1) Etape d'agglutination avec une lectine telle que par exemple Phaseolus vulgaris,
2) Etape de lyse cellulaire avec une combinaison de détergents tels que saponine, Tween 20 et Triton X 100.

Le marquage et la perméabilisation des germes pathogènes pouvant intervenir indistinctement lors de l'étape d'agrégation ou lors de l'étape de lyse.

## Revendications

1. Procédé de détection de germes contaminants éventuellement présents dans un produit sanguin comprenant des cellules sanguines, **caractérisé en ce qu'**il comprend les étapes suivantes :
a) on soumet un échantillon dudit produit sanguin à un traitement d'agrégation des cellules sanguines,
b) on élimine les agrégats formés à l'étape (a) par passage de l'échantillon traité sur un premier filtre laissant passer les germes contaminants mais pas les agrégats de cellules,
c) on lyse sélectivement les cellules résiduelles du filtrat obtenu à l'étape (b),
d) on récupère les germes contaminants par passage du lysat de l'étape (c) sur un second filtre laissant passer les débris cellulaires,
e) on ajoute un agent de marquage des germes contaminants, soit lors de l'agrégation de l'étape (a), soit lors de la lyse de l'étape (c), et
f) on analyse le second filtre pour détecter les germes contaminants marqués éventuellement retenus.

2. Procédé selon la revendication 1, **caractérisé en ce qu'**il comprend l'addition d'un agent de perméabilisation des germes contaminants, à au moins l'une des étapes (a), (c), ou (e).

3. Procédé selon la revendication 2, **caractérisé en ce que** l'agent de perméabilisation est choisi dans le groupe comprenant : la polyéthylènimine, le diacétate de chlorhexidine, le digluconate de chlorexhidine, l'acide d'éthylène diamine tetracétate (EDTA) seul ou en combinaison avec la nisine, un détergent ou un mélange de ceux-ci.

4. Procédé selon la revendication 3, **caractérisé en ce que** le détergent est choisi parmi le groupe comprenant : N-Octyl β-D-glucopyranoside, SDS, Tween, triton, Brij, ou un mélange de ceux-ci.

5. Procédé selon l'une quelconque des revendications 2 à 4, **caractérisé en ce que** l'agent de marquage des germes pathogènes est une solution de marquage choisie parmi le groupe comprenant un substrat estérasique tel que le ChemChrome V6, un anticorps marqué ou un marqueur d'acides nucléiques.

6. Procédé selon la revendication 5, **caractérisé en ce que** l'agent de marquage des germes pathogènes est un marqueur d'acides nucléiques.

7. Procédé selon l'une quelconque des revendications 2 à 6, **caractérisé en ce que** l'agent de marquage comprend un marqueur fluorescent ou un agent couplé à un fluorochrome ou à un enzyme permettant la dégradation d'un substrat rendu ainsi fluorescent.

8. Procédé selon la revendication 7, **caractérisé en ce que** la fluorescence est détectée au moyen d'une excitation laser.

9. Procédé selon l'une quelconque des revendications 1 à 4, **caractérisé en ce que** les cellules sanguines du produit sanguin sont des plaquettes ou des globules rouges ou un mélange de ceux-ci.

10. Procédé selon l'une quelconque des revendications 1 à 9, **caractérisé en ce que** le produit sanguin comprend des plaquettes et **en ce que** le traitement d'agrégation de l'étape (a) comprend la mise en contact de l'échantillon avec une composition d'agrégation comprenant au moins l'un des agents d'agrégation choisis dans le groupe comprenant :
- un anticorps spécifique d'un antigène plaquettaire,
- un agoniste fort de l'activation plaquettaire choisi parmi : la thrombine, le TRAP (thrombin receptor activating peptide), la trypsine, le collagène, le thromboxane A2, le PAF (platelet activating factor), le ionophore A23187, les complexes immuns et facteurs du complément.
- un agoniste faible de l'activation plaquettaire choisi parmi ADP, adrénaline, acide arachidonique, facteur Von Willebrand, sérotonine ou épinéphrine.

11. Procédé selon la revendication 10, **caractérisé en ce que** la concentration en anticorps de type CD9 dans la composition d'agrégation est comprise entre 0,5 *µ*g/ml et 50 *µ*g/ml, et de préférence entre 5 *µ*g/ml et 40 *µ*g/ml.

12. Procédé selon la revendication 10, **caractérisé en ce que** la concentration en agoniste fort dans la composition d'agrégation est comprise entre :
- 0,5 UI/ml et 100 UI/ml et de préférence entre 1 UI/ml et 20 UI/ml pour un agoniste de type thrombine,
- 5 µM et 200 µM et de préférence entre 10 µM et 100 µM pour un agoniste de type TRAP,
- 1 nM et 500 nM et de préférence entre 10 nM et 300 nM pour un agoniste de type trypsine,
- 0,05 µg/ml et 50 µg/ml et de préférence entre 1 µg/ml et 20 µg/ml pour un agoniste de type collagène,
- 0,01 µg/ml et 5 µg/ml et de préférence entre 0,1 µg/ml et 1 µg/ml pour un agoniste de type thromboxane A2,
- 0,005 mg/ml et 1 mg/ml et de préférence entre 0,05 mg/ml et 0,5 mg/ml pour un agoniste de type PAF,
- 0,1 µM et 100 µM et de préférence entre 1 µM et 20 µM pour un agoniste de type ionophore A23187.

13. Procédé selon la revendication 10, **caractérisé en ce que** la concentration en agoniste faible dans la composition d'agrégation est comprise entre :
- 0,5 µM et 100 µM et de préférence entre 1 µM et 20 µM pour un agoniste du type ADP, du typa adrénaline ou du type epinéphrine,
- 0,001 mM et 10 mM et de préférence entre 0,01 mM et 5 mM pour un agoniste du type acide arachidonique,
- 0,001 mg/ml et 1 mg/ml et de préférence entre 0,01 mg/ml et 0,5 mg/ml pour un agoniste du type facteur Von Willerbrand, ou
- 0,05 µM et 100 µM et de préférence entre 0,01 µM et 50 µM pour un agoniste du type sérotonine.

14. Procédé selon la revendication 10, **caractérisé en ce que** l'anticorps est choisi parmi : un anticorps anti-CD9, anti-CD32, anti-PTA1, anti-CD42, anti-GpIIb/IIIa ou anti-GpIV.

15. Procédé selon l'une quelconque des revendications 1 à 14, **caractérisé en ce que** le produit sanguin comprend des globules rouges et **en ce que** le traitement d'agrégation de l'étape (a) comprend la mise en contact de l'échantillon avec une composition d'agglutination comprenant au moins un agent d'agglutination choisi parmi les lectines, le polyéthylènimine, la polyvinylpyrrolidone (PVP), les gélatines, les dextrans ou les polyéthylène glycols (PEG).

16. Procédé selon la revendication 15, **caractérisé en ce que** les lectines présentent une activité érythroagglutinine.

17. Procédé selon la revendication 15 ou 16, **caractérisé en ce que** les lectines sont choisies parmi les lectines de *Phaseolus vulgaris, Vicia sativa, Vicia faba* ou *Erythrina corallodendron.*

18. Procédé selon la revendication 17, **caractérisé en ce que** la concentration de lectine de type *Phaseolus vulgaris* dans la composition d'agglutination est comprise entre 10 µg/ml et 200 µg/ml.

19. Procédé selon la revendication 15, **caractérisé en ce que** la concentration de polyéthylènimine dans la composition d'agglutination est comprise entre 0,1% (poids/volume) et 40% (poids/volume), de préférence entre 20% (poids/volume) et 40% (poids/volume).

20. Procédé selon la revendication 15, **caractérisé en ce que** la polyvinylpyrrolidone (PVP) est choisie parmi le groupe comprenant du PVP-40 et du PVP-360 et utilisée de préférence à une concentration comprise entre 0,1% (poids/volume) et 40% (poids/volume), et tout préférentiellement entre 4% (poids/volume) et 20% (poids/volume).

21. Procédé selon la revendication 15, **caractérisé en ce que** la gélatine dans la composition d'agglutination est utilisée à une concentration comprise entre 0,5% (poids/volume) et 40% (poids/volume), de préférence entre 4% (poids/volume) et 20% (poids/volume).

22. Procédé selon la revendication 15, **caractérisé en ce que** le dextran est dans la composition d'agglutination est choisi parmi le groupe comprenant : Dextran 70, Dextran 100, et Dextran 500, utilisé de préférence à une concentration comprise entre 0,1% (poids/volume) et 40% (poids/volume), et tout préférentiellement entre 4% (poids/volume) et 20% (poids/volume).

23. Procédé selon la revendication 15, **caractérisé en ce que** le polyéthylène glycol est choisi parmi le groupe comprenant : PEG8, PEG17, PEG35, utilisé de préférence à une concentration comprise entre 0,05% (poids/volume) et 40% (poids/volume), et tout préférentiellement entre 20% (poids/volume) et 40% (poids/volume).

24. Procédé selon l'une quelconque des revendications précédentes, **caractérisé en ce que** la lyse des cellules de l'étape (c) est réalisée avec une solution de lyse comprenant un ou plusieurs détergents choisis dans le groupe comprenant : saponine, SDS, Tween 20, Triton X100, Brij 96, Polidocanol, ou Carbonate de sodium.

25. Procédé selon l'une quelconque des revendications précédentes, **caractérisé en ce que** les germes contaminants sont des bactéries aérobies ou anaérobies, des moisissures, des levures, des spores de bactéries vivantes et/ou mortes.

26. Procédé selon l'une quelconque des revendications précédentes, **caractérisé en ce que** la taille des pores du premier filtre est comprise entre 2 µm et 20 µm.

27. Procédé selon l'une quelconque des revendications précédentes, **caractérisé en ce que** la taille des pores du second filtre est comprise entre 0,2 µm et 2 µm.

28. Procédé selon l'une quelconque des revendications précédentes, **caractérisé en ce que** les germes contaminants sont des bactéries aérobies ou anaérobies, des moisissures, des levures ou des spores de bactéries vivantes et/ou mortes , **en ce que** la taille des pores du premier filtre est comprise entre 2 µm et 20 µm, et **en ce que** la taille des pores du second filtre est comprise entre 0,2 µm et 2 µm.

29. Procédé selon l'une quelconque des revendications 2 à 28, **caractérisé en ce que** la détection des germes contaminants de l'étape (f) est réalisée dans un dispositif clos.

## Claims

1. Method for detecting contaminating germs potentially present in a blood product containing blood cells, **characterised in that** it includes the following steps:
a) subjecting a sample of said blood product to a blood cell aggregation treatment,
b) eliminating the aggregates formed at step (a) by passing the treated sample over a first filter allowing the contaminating germs to pass through but not the cell aggregates,
c) selectively lyzing the residual cells of the filtrate obtained at step (b),
d) recovering the contaminating germs by passing the lysate of step (c) over a second filter allowing the cell debris to pass through,
e) adding a contaminating germ-labelling agent either during the aggregation step (a), or during the lysis of step (c), and
(f) examining the second filter in order to detect the labelled contaminating germs possibly retained.

2. Method as claimed in claim 1, **characterised in that** it includes the addition of a contaminating germ-permeabilization agent, during at least one of the steps (a), (c) or (e).

3. Method as claimed in claim 2, **characterised in that** the permeabilization agent is chosen from the group including: polyethylene imine, chlorexhidine diacetate, chlorhexidine digluconate, ethylenediaminetetraacetic acid (EDTA) alone or in combination with nisin, a detergent or a mixture thereof.

4. Method as claimed in claim 3, **characterised in that** the detergent is chosen from among the group including: n-Octyl-β-D-glucopyranoside, SDS, Tween, Triton, Brij, or a mixture thereof.

5. Method as claimed in any of claims 2 to 4, **characterised in that** the pathogenic germ-labelling agent is a labelling solution chosen from among the group including an esterase substrate such as ChemChrome V6, a labelled antibody or a nucleic acid label.

6. Method as claimed in claim 5, **characterised in that** the pathogenic germ-labelling agent is a nucleic acid label.

7. Method as claimed in any of claims 2 to 6, **characterised in that** the labelling agent includes a fluorescent label or an agent coupled to a fluorochrome or to an enzyme enabling the degradation of a substrate thereby rendered fluorescent.

8. Method as claimed in claim 7, **characterised in that** the fluorescence is detected by means of laser excitation.

9. Method as claimed in any of claims 1 to 4, **characterised in that** the blood cells of the blood product are platelets or red blood cells or a mixture thereof.

10. Method as claimed in any of claims 1 to 9, **characterised in that** the blood product contains platelets and **in that** the aggregation treatment of step (a) includes placing the sample in contact with an aggregation composition containing at least one of the aggregation agents chosen from the group including:
- a platelet antigen-specific antibody,
- a strong platelet activation agonist chosen from among: thrombin, TRAP (thrombin receptor-activating peptide), trypsin, collagen, thromboxane A2, PAF (platelet activating factor), ionophore A23187, immune complexes and complement components,
- a weak platelet activation agonist chosen from among ADP, adrenalin, arachidonic acid, Von Willebrand factor, serotonin or epinephrine.

11. Method as claimed in claim 10, **characterised in that** the type CD9 antibody concentration in the aggregation composition is between 0.5 µg/ml and 50 µg/ml, and preferably between 5 µg/ml and 40 µg/ml.

12. Method as claimed in claim 10, **characterised in that** the strong agonist concentration in the aggregation composition is between:
- 0.5 UI/ml and 100 UI/ml and preferably between 1 UI/ml and 20 UI/ml for a thrombin-type agonist,
- 5 µM and 200 µM and preferably between 10 µM and 100 µM for a TRAP-type agonist,
- 1 nM and 500 nM and preferably between 10 nM and 300 nM for a trypsin-type agonist,
- 0.05 µg/ml and 50 µg/ml and preferably between 1 µg/ml and 20 µg/ml for a collagen-type agonist,
- 0.01 µg/ml and 5 µg/ml and preferably between 0.1 µg/ml and 1 µg/ml for a thromboxane A2-type agonist,
- 0.005 mg/ml and 1 mg/ml and preferably between 0.05 mg/ml and 0.5 mg/ml for a PAF-type agonist,
- 0.1 µM and 100 µM and preferably between 1 µM and 20 µM for an ionophore A23187-type agonist.

13. Method as claimed in claim 10, **characterised in that** the weak agonist concentration in the aggregation composition is between:
- 0.5 µM and 100 µM and preferably between 1 µM and 20 µM for an ADP-type agonist, of the adrenalin or epinephrine type,
- 0.001 mM and 10 mM and preferably between 0.01 mM and 5 mM for an arachidonic acid-type agonist,
- 0.001 mg/ml and 1 mg/ml and preferably between 0.01 mg/ml and 0.5 mg/ml for a Von Willerbrand factor-type agonist, or
- 0.05 µM and 100 µM and preferably between 0.01 µM and 50 µM for a serotonin-type agonist.

14. Method as claimed in claim 10, **characterised in that** the antibody is chosen from among: an anti-CD9, anti-CD32, anti-PTA1, anti-CD42, anti-GPIIb/IIIa or anti-GPIV antibody.

15. Method as claimed in any of claims 1 to 14, **characterised in that** the blood product contains red blood cells and **in that** the aggregation treatment of step (a) includes placing the sample in contact with an agglutination composition containing at least one agglutination agent chosen from among lectins, polyethylene imine, polyvinylpyrrolidone (PVP), gelatins, dextrans or polyethylene glycols (PEG).

16. Method as claimed in claim 15, **characterised in that** the lectins have an erythroagglutinin activity.

17. Method as claimed in claim 15 or 16, **characterised in that** the lectins are chosen from among lectins of *Phaseolus vulgaris, Vicia sativa, Vicia faba* or *Erythrina corallodendron.*

18. Method as claimed in claim 17, **characterised in that** the concentration of *Phaseolus vulgaris*-type lectin in the agglutination composition is between 10 µg/ml and 200 µg/ml.

19. Method as claimed in claim 15, **characterised in that** the polyethylene imine concentration in the agglutination composition is between 0.1% (weight/volume) and 40% (weight/volume), preferably between 20% (weight/volume) and 40% (weight/volume).

20. Method as claimed in claim 15, **characterised in that** the polyvinylpyrrolidone (PVP) is chosen from among the group including PVP-40 and PVP-360 and is preferably used at a concentration of between 0.1% (weight/volume) and 40% (weight/volume), and most preferably between 4% (weight/volume) and 20% (weight/volume).

21. Method as claimed in claim 15, **characterised in that** the gelatin in the agglutination composition is between 0.5% (weight/volume) and 40% (weight/volume), preferably between 4% (weight/volume) and 20% (weight/volume).

22. Method as claimed in claim 15, **characterised in that** the dextran is in the agglutination composition is chosen from among the group including: Dextran 70, Dextran 100, and Dextran 500, preferably used at a concentration of between 0.1% (weight/volume) and 40% (weight/volume), and most preferably between 4% (weight/volume) and 20% (weight/volume).

23. Method as claimed in claim 15, **characterised in that** the polyethylene glycol is chosen from among the group including: PEG8, PEG17, PEG35, preferably used at a concentration of between 0.05% (weight/volume) and 40% (weight/volume) and most preferably between 20% (weight/volume) and 40% (weight/volume).

24. Method as claimed in any of the preceding claims, **characterised in that** the cell lysis of step (c) is carried out with a lysis solution containing one or more detergents chosen from among the group including: saponin, SDS, Tween 20, Triton X100, Brij 96, Polidocanol, or sodium carbonate.

25. Method as claimed in any of the preceding claims, **characterised in that** the contaminating germs are aerobic or anaerobic bacteria, moulds, yeasts, spores of living and/or dead bacteria.

26. Method as claimed in any of the preceding claims, **characterised in that** the pore size of the first filter is between 2 µm and 20 µm.

27. Method as claimed in any of the preceding claims, **characterised in that** the pore size of the second filter is between 0.2 µm and 2 µm.

28. Method as claimed in any of the preceding claims, **characterised in that** the contaminating germs are aerobic or anaerobic bacteria, moulds, yeasts or spores of living and/or dead bacteria, **in that** the pore size of the first filter is between 2 µm and 20 µm, and **in that** the pore size of the second filter is between 0.2 µm and 2 µm.

29. Method as claimed in any of claims 2 to 28, **characterised in that** the detection of the contaminating germs of step (f) is carried out in a sealed device.

## Patentansprüche

1. Verfahren zum Nachweis eventuell in einem Blutprodukt vorhandener kontaminierender Keime, Blutzellen umfassend, **dadurch gekennzeichnet, dass** es die folgenden Schritte umfasst:
a) man unterzieht eine Probe des besagten Blutprodukts einer Aggregationsbehandlung der Blutzellen,
b) man entfernt die in Schritt (a) gebildeten Aggregate durch Passage der behandelten Probe über einen ersten Filter, der die kontaminierenden Keime, aber nicht die Zellaggregate passieren lässt,
c) man lysiert selektiv die residuellen Zellen des in Schritt (b) gewonnenen Filtrats,
d) man gewinnt die kontaminierenden Keime durch Passage des Lysats von Schritt (c) über einen zweiten Filter zurück, der den Zellschrott passieren lässt,
e) man fügt einen Marker für die kontaminierenden Keime hinzu, entweder bei der Aggregation von Schritt (a) oder bei der Lyse von Schritt (c), und
f) man analysiert den zweiten Filter, um die eventuell zurückgehaltenen markierten kontaminierenden Keime nachzuweisen.

2. Verfahren nach Anspruch 1, **dadurch gekennzeichnet, dass** es mindestens bei einem der Schritte (a), (c) oder (e) die Addition eines Permeabilisierungsmittels für die kontaminierenden Keime umfasst

3. Verfahren nach Anspruch 2, **dadurch gekennzeichnet, dass** das Permeabilisierungsmittel ausgewählt ist aus der Gruppe, die das Polyethylenimin, das Chlorhexidindiacetat, das Chlorhexidindigluconat, die Ethylendiamintetraessigsäure (EDTA) allein oder in Kombination mit dem Nisin, einem Detergens oder einem Gemisch derselben umfasst.

4. Verfahren nach Anspruch 3, **dadurch gekennzeichnet, dass** das Detergens ausgewählt ist aus der Gruppe, die N-Octyl-β-D-glucopyranosid, SDS, Tween, Triton, Brij oder ein Gemisch derselben umfasst.

5. Verfahren nach einem der Ansprüche 2 bis 4, **dadurch gekennzeichnet, dass** der Marker der pathogenen Keime eine Markerlösung ist, die ausgewählt ist aus der Gruppe, die ein esterasisches Substrat wie ChemChrome V6, einen markierten Antikörper oder einen Nukleinsäuremarker umfasst.

6. Verfahren nach Anspruch 5, **dadurch gekennzeichnet, dass** der Marker der pathogenen Keime ein Nukleinsäuremarker ist.

7. Verfahren nach einem der Ansprüche 2 bis 6, **dadurch gekennzeichnet, dass** der Marker ein fluoreszierender Marker oder ein Mittel ist, das an ein Fluorochrom oder an ein Enzym gekoppelt ist, das den Abbau eines auf die Art fluoreszierend gewordenen Substrats erlaubt.

8. Verfahren nach Anspruch 7, **dadurch gekennzeichnet, dass** die Fluoreszenz mittels einer Laserexzitation nachgewiesen wird.

9. Verfahren nach einem der Ansprüche 1 bis 4, **dadurch gekennzeichnet, dass** die Blutzellen des Blutprodukts Blutplättchen oder rote Blutkörperchen oder ein Gemisch derselben sind.

10. Verfahren nach einem der Ansprüche 1 bis 9, **dadurch gekennzeichnet, dass** das Blutprodukt Blutplättchen umfasst und dadurch, dass die Aggregationsbehandlung von Schritt (a) die Herstellung eines Kontakts der Probe mit einer Aggregationszusammensetzung umfasst, die mindestens eines der Aggregationsmittel umfasst, die ausgewählt sind aus der Gruppe, die umfasst:
- einen speziellen Antikörper eines Blutplättchenantigens,
- einen starken Agonisten der Blutplättchenaktivierung, der aus dem Thrombin, dem TRAP (Thrombin Rezeptor aktivierendes Peptid), dem Trypsin, dem Kollagen, dem Thromboxan A2, dem PAF (platelet activating factor), dem Ionophor A23187, dem Immunkomplexen und -faktoren des Komplements ausgewählt ist,
- einen schwachen Agonisten der Blutplättchenaktivierung, der aus ADP, Adrenalin, Arachidonsäure, von Willebrand-Faktor, Serotonin oder Epinephrin ausgewählt ist.

11. Verfahren nach Anspruch 10, **dadurch gekennzeichnet, dass** die Antikörperkonzentration vom Typ CD9 in der Aggregationszusammensetzung zwischen 0,5 µg/ml und 50 µ g/ml inklusive und vorzugsweise zwischen 5 µg/ml und 40 µg/ml beträgt.

12. Verfahren nach Anspruch 10, **dadurch gekennzeichnet, dass** die Konzentration des starken Agonisten in der Aggregationszusammensetzung beträgt zwischen inklusive:
- 0,5 UI/ml und 100 UI/ml und vorzugsweise zwischen 1 UI/ml und 20 UI/ml für einen Agonisten vom Typ Thrombin,
- 5 µM und 200 µM und vorzugsweise zwischen 10 µM und 100 µM für einen Agonisten vom Typ TRAP,
- 1 nM und 500 nM und vorzugsweise zwischen 10 nM und 300 nM für einen Agonisten vom Typ Trypsin,
- 0,05 µg/ml und 50 µg/ml und vorzugsweise zwischen 1 µ g/ml und 20 µg/ml für einen Agonisten vom Typ Kollagen,
- 0,01 µg/ml und 5 µg/ml und vorzugsweise zwischen 0,1 µg/ml und 1 µg/ml für einen Agonisten vom Typ Thromoxan A2,
- 0,005 mg/ml und 1 mg/ml und vorzugsweise zwischen 0,05 mg/ml und 0,5 mg/ml für einen Agonisten vom Typ PAF,
- 0,1 µM und 100 µM und vorzugsweise zwischen 1 µM und 20 µM für einen Agonisten vom Typ Ionophor A23187.

13. Verfahren nach Anspruch 10, **dadurch gekennzeichnet, dass** die Konzentration des schwachen Agonisten in der Aggregationszusammensetzung beträgt zwischen inklusive:
- 0,5 µM und 100 µM und vorzugsweise zwischen 1 µM und 20 µM für einen Agonisten vom Typ ADP, vom Typ Adrenalin oder vom Typ Epinephrin,
- 0,001 mM und 10 mM und vorzugsweise zwischen 0,01 mM und 5 mM für einen Agonisten vom Typ Arachidonsäure,
- 0,001 mg/ml und 1 mg/ml und vorzugsweise zwischen 0,01 mg/ml und 0,5 mg/ml für einen Agonisten vom Typ von Willebrand-Faktor, oder
- 0,05 µM und 100 µM und vorzugsweise zwischen 0,01 µM und 50 µM für einen Agonisten vom Typ Serotonin.

14. Verfahren nach Anspruch 10, **dadurch gekennzeichnet, dass** der Antikörper aus einem Antikörper Anti-CD9, Anti-CD32, Anti-PTA1, Anti-CD42, Anti-GpIIb/IIIa oder Anti-GpIV ausgewählt ist.

15. Verfahren nach einem der Ansprüche 1 bis 14, **dadurch gekennzeichnet, dass** das Blutprodukt rote Blutkörperchen umfasst und dadurch, dass die Aggregationsbehandlung von Schritt (a) die Herstellung eines Kontakts der Probe mit einer Agglutinierungszusammensetzung umfasst, die mindestens ein Agglutinierungsmittel umfasst, das aus den Lektinen, dem Polyethylenimin, dem Polyvinylpyrrolidon (PVP), den Gelatinen, den Dextranen oder den Polyethylenglycolen (PEG) ausgewählt ist.

16. Verfahren nach Anspruch 15, **dadurch gekennzeichnet, dass** die Lektine eine Erythrozyten agglutinierende Aktivität aufweisen.

17. Verfahren nach Anspruch 15 oder 16, **dadurch gekennzeichnet, dass** die Lektine aus den Lektinen *Phaseolus vulgaris, Vicia sativa, Vicia faba* oder *Erythrina corallodendron* ausgewählt sind.

18. Verfahren nach Anspruch 17, **dadurch gekennzeichnet, dass** die Lektinkonzentration vom Typ *Phaseolus vulgaris* in der Agglutinierungszusammensetzung zwischen inklusive 10 µg/ml und 200 µg/ml beträgt.

19. Verfahren nach Anspruch 15, **dadurch gekennzeichnet, dass** die Polyethyleniminkonzentration in der Agglutinierungszusammensetzung zwischen inklusive 0,1 % (Gewicht/Volumen) und 40 %, vorzugsweise zwischen 20 % (Gewicht/Volumen) und 40 % (Gewicht/Volumen), beträgt.

20. Verfahren nach Anspruch 15, **dadurch gekennzeichnet, dass** das Polyvinylpyrrolidon (PVP) aus der Gruppe ausgewählt ist, die PVP-40 und PVP-360 umfasst und vorzugsweise bei einer Konzentration zwischen inklusive 0,1 % (Gewicht/Volumen) und 40 % (Gewicht/Volumen) und sehr bevorzugt zwischen 4 % (Gewicht/Volumen) und 20 % (Gewicht/Volumen) verwendet wird.

21. Verfahren nach Anspruch 15, **dadurch gekennzeichnet, dass** die Gelatine in der Agglutinierungszusammensetzung bei einer Konzentration zwischen inklusive 0,5 % (Gewicht/Volumen) und 40 % (Gewicht/Volumen) und vorzugsweise zwischen 4 % (Gewicht/Volumen) und 20 % (Gewicht/Volumen) verwendet wird.

22. Verfahren nach Anspruch 15, **dadurch gekennzeichnet, dass** das Dextran in der Agglutinierungszusammensetzung aus der Gruppe ausgewählt ist, die Dextran 70, Dextran 100 und Dextran 500 umfasst, verwendet vorzugsweise bei einer Konzentration zwischen inklusive 0,1 % (Gewicht/Volumen) und 40 % (Gewicht/Volumen) und sehr bevorzugt zwischen 4 % (Gewicht/Volumen) und 20 % (Gewicht/Volumen).

23. Verfahren nach Anspruch 15, **dadurch gekennzeichnet, dass** das Polyethylenglycol aus der Gruppe ausgewählt ist, die PEG8, PEG17, PEG35 umfasst, verwendet vorzugsweise bei einer Konzentration zwischen inklusive 0,05 % (Gewicht/Volumen) und 40 % (Gewicht/Volumen) und sehr bevorzugt zwischen 20 % (Gewicht/Volumen) und 40 % (Gewicht/Volumen).

24. Verfahren nach einem der vorangehenden Ansprüche, **dadurch gekennzeichnet, dass** die Zelllyse von Schritt (c) mit einer Lyselösung durchgeführt wird, die ein Detergens oder mehrere Detergenzien umfasst, die aus der Gruppe ausgewählt sind, die Saponin, SDS, Tween 20, Triton X100, Brij 96, Polidocanol oder Natriumcarbonat umfasst.

25. Verfahren nach einem der vorangehenden Ansprüche, **dadurch gekennzeichnet, dass** die kontaminierenden Keime aerobe oder anaerobe Bakterien, Pilze, Hefen, Sporen lebender und/oder toter Bakterien sind.

26. Verfahren nach einem der vorangehenden Ansprüche, **dadurch gekennzeichnet, dass** die Größe der Poren des ersten Filters zwischen inklusive 2 µm und 20 µm beträgt.

27. Verfahren nach einem der vorangehenden Ansprüche, **dadurch gekennzeichnet, dass** die Größe der Poren des zweiten Filters zwischen inklusive 0,2 µm und 2 µm beträgt.

28. Verfahren nach einem der vorangehenden Ansprüche, **dadurch gekennzeichnet, dass** die kontaminierenden Keime aerobe oder anaerobe Bakterien, Pilze, Hefen, Sporen lebender und/oder toter Bakterien sind, und dadurch, dass die Größe der Poren des ersten Filters zwischen inklusive 2 µm und 20 µm beträgt und dadurch, dass die Größe der Poren des zweiten Filters zwischen inklusive 0,2 µm und 2 µm beträgt.

29. Verfahren nach einem der Ansprüche 2 bis 28, **dadurch gekennzeichnet, dass** der Nachweis der kontaminierenden Keime von Schritt (f) in einer geschlossenen Vorrichtung erfolgt.
